# EUROPEAN PATENT APPLICATION

(11) **EP 4 617 651 A1**
(43) Date of publication of application: **17.09.2025**
(21) Application number: 24197027.6
(22) Date of filing: 28.08.2024
(51) Int. Cl.: G01N 27/66

(54) **PHOTOIONIZATION DETECTOR WITH IGNITION FAILURE WARNING SYSTEM**

(30) Priority: 15.03.2024 US 202463565765 P
(71) Applicant: Mocon, Inc., Minneapolis, Minnesota 55428 (US)
(72) Inventor: Willcox, Charles, Minneapolis, 55428 (US); Jennings, David, Minneapolis, 55428 (US); Fields, Christopher, Minneapolis, 55428 (US)
(74) Representative: Vossius & Partner Patentanwälte Rechtsanwälte mbB

(57) **Abstract**

A photoionization (PID) detector with an ignition failure warning system. The PID detector includes an ultraviolet (UV) lamp, sensing electrodes, ignition electrodes, a driver, an ignition failure warning electrical circuit, and a processor. The ignition failure warning electrical circuit is operable for detecting an absence of ultraviolet radiation emitted by the ultraviolet lamp after an attempted ignition of the ultraviolet lamp by the driver, and generating an uncharacteristically dramatic human perceptible high and low oscillation of reported concentrations of target analyte upon detecting an absence of ultraviolet radiation emitted by the ultraviolet lamp after an attempted ignition of the ultraviolet lamp by the driver.

## Description

### BACKGROUND

Photoionization detector (PID) sensors break molecules, typically volatile organic molecules, into positively charged ions and free electrons using high-energy ultraviolet (UV) radiation emitted by a UV lamp. As the target analyte enters the detector, it is bombarded by high-energy UV photons. The target analyte absorbs the UV light, resulting in ejection of electrons and the formation of positively charged ions. The positively charged ions are sensed by an anode/cathode arrangement in the detector and produce an electric current whose amplitude is proportional to the positively charged ions produced (*i.e.,* the greater the concentration of target analyte in the sample entering the detector, the more positively charged ions produced, resulting in a greater current). The current is amplified, displayed on an ammeter, or converted via an algorithm or a look-up table to a concentration of target molecules and digitally displayed.

UV lamps are ignited by introduction of an electrical discharge into the gaseous content of the lamp (typically Krypton) by a driver and a pair of ignition electrodes. However, not every ignition attempt is successful. A failure to ignite is always possible and tends to increase under a variety of conditions and for a variety of reasons including aging of the UV lamp, loss of gaseous content from the UV lamp, and cold environmental conditions.

Ignition reliability can be increased by exposing the gaseous content of the UV lamp to blue wavelength electromagnetic radiation, typically via a blue LED capable of emitting electromagnetic radiation at wavelengths that include those at 400 to 500 nm, prior to an ignition attempt. The blue wavelength electromagnetic radiation can ionize a small quantity of the gas molecules within the lamp, thereby increasing the chances of achieving ignition - especially at low temperatures. While effective as an ignition assist, use of an ignition assist light source such as a blue LED is not always effective and suffers the drawback of significantly decreasing the useful life of battery powered PID sensors as the ignition assist light source remains on and draws power throughout an entire testing period.

Failure to recognize the failed ignition of a UV lamp in a PID sensor can result in lost time when testing continues even though the lamp has failed to ignite, and can even result in an erroneous and potentially hazardous reading when ignition failure remains unnoticed and readings of 0% target analyte are accepted as accurate. It is therefore desirable to know when an attempted ignition was unsuccessful. This is particularly true when the PID sensor is employed in situations where timely reporting of potentially hazardous concentrations of target analyte is a safety requirement.

A known method employed to alert a user to a failed ignition detects a failed ignition and reports the failure as a low or zero target analyte concentration. However, this method is unsuited and unreliable when the PID sensor is used in situations where low or ultra-low concentrations of target analyte are being measured and/or situations where higher concentrations of target analyte are expected to be encountered, but only on a sporadic basis with low or near zero concentrations encountered in the interim between events. A user will be unable to consistently distinguish between reporting of a failed ignition and reporting of an actual low or zero target analyte concentration.

Hence, a need exists for a reliable means of detecting and unambiguously reporting unsuccessful attempts to ignite the UV lamp in a PID sensor. It is further desired to achieve such detection without deviating from the customary three terminals used with PID sensor, one for input voltage, a second for ground and a third for transmission of output signal proportional to the sensed concentration of target analyte. It is further desired to achieve such detection even when electromagnetic radiation from an ignition assist light source is observed, and even more desired to achieve detection with concomitant activation of the ignition assist light source only when an unsuccessful ignition is detected.

### SUMMARY OF THE INVENTION

The invention is a photoionization (PID) detector with an ignition failure warning system. The PID detector includes (i) an ultraviolet (UV) lamp, (ii) sensing electrodes, (iii) ignition electrodes, (iv) a driver, (v) an ignition failure warning electrical circuit, and (vi) a processor, each as detailed below.

The UV lamp is operable when ignited for emitting ultraviolet radiation effective to ionize a target analyte, such as a volatile organic solvent within a sample. The sensing electrodes are operable for detecting the presence of ionized target analyte within the sample and generating a genuine value electrical signal having a value proportional to the concentration of target analyte within the sample. The ignition electrodes are operable for igniting the ultraviolet lamp. The driver supplies an ignition current to the ignition electrodes, theoretically effective to ignite the ultraviolet lamp. The ignition failure warning electrical circuit is operable for (A) detecting an absence of ultraviolet radiation emitted by the ultraviolet lamp after an attempted ignition of the ultraviolet lamp by the driver, and (B) generating alternating high value and low value secondary electrical signals upon detecting an absence of ultraviolet radiation emitted by the ultraviolet lamp after an attempted ignition of the ultraviolet lamp by the driver. The processor is operable for receiving the genuine value electrical signal and the alternating high value and low value secondary signals, and generating a human perceptible indication of failed ignition when the secondary signals are received.

The photoionization (PID) detector can include a housing defining a sample retention chamber for enclosing the sample to be tested by the photoionization (PID) detector.

The sensing electrodes can be an anode-cathode pair.

The human perceptible indication of failed ignition generated by the processor can be a human perceptible oscillation of reported concentrations of target analyte.

In a preferred embodiment, the photoionization (PID) detector further includes a selectively activatable ignition assist light source different from the ultraviolet lamp for emitting electromagnetic radiation ineffective for detectably ionizing a target analyte within a sample, and the ignition failure warning system is an electrical circuit operable for (A) detecting an absence of ultraviolet radiation emitted by the ultraviolet lamp after an attempted ignition of the ultraviolet lamp by the driver, (B) oscillating powering of the ignition assist light source as between a light-on status and light-off status upon detecting an absence of ultraviolet radiation emitted by the ultraviolet lamp after an attempted ignition of the ultraviolet lamp by the driver, (C) detecting the on-off status of the ignition assist light source over time, and (D) generating a light-on electrical signal having a value when the ignition assist light source is on and generating a light-off electrical signal having a value different from the value of the light-on electrical signal when the ignition assist light source is off, so as to generate an oscillating set of high value and low value secondary electrical signals.

In further detail, the ignition failure warning electrical circuit can include (i) a photodiode, (ii) a transimpedance amplifier, (iii) a low pass filter, and (iv) a comparator with hysteresis, each as detailed below.

The photodiode is operable for (A) periodically detecting presence and absence of radiation emitted by at least one of the ultraviolet lamp or the ignition assist light source after an attempted ignition of the ultraviolet lamp by the driver, and (B) periodically generating an ON current signal when the presence of ultraviolet radiation emitted by at least one of the ultraviolet lamp and the light source is detected, and otherwise bypassing generation of an ON signal.

The transimpedance amplifier is in electrical communication with the photodiode and operable for receiving and converting each ON current signal to an ON voltage signal and in the absence of receiving an ON current signal bypassing generation of an ON voltage signal.

The low pass filter has a time constant (*e.g*., between 0.1 and 10 Hz and preferably between about 0.5 to 5 Hz) in electrical communication between the transimpedance amplifier, the ignition assist light source and the processor for receiving each ON voltage signal and providing a timed passage of each ON voltage signal in accordance with the time constant.

The comparator is in electrical communication with the low pass filter for (A) receiving each ON voltage signal and generating a first electrical output when each ON voltage signal is received operable for powering off the ignition assist light source and transmitting the low value secondary electrical signal to the processor, and (B) generating a second electrical output when no ON voltage signal is received operable for powering on the ignition assist light source and transmitting the high value secondary electrical signal to the processor.

### BRIEF DESCRIPTION OF THE DRAWINGS

Figure 1 is a schematic view of one embodiment of a typical photoionization detector (PID) sensor with ignition assist light.
Figure 2 is a circuit diagram of a PID sensor with one embodiment of an ignition failure warning system in accordance with the invention.
Figure 3 is a generalized flowchart for operation of a typical photoionization detector (PID) sensor with ignition assist light equipped with an ignition failure warning system in accordance with the invention.

### DETAILED DESCRIPTION OF A PREFERRED EMBODIMENT

### Definitions

As utilized herein, including the claims, the terms "***high value***" and "***low value***", when used to modify the noun "secondary electrical signal", are relative terms referencing values that are higher and lower relative to one another, with a "high value" having a value greater than a "low value". The terms "high value" and "low value" do not indicate any particular numerical value nor any particular difference (*i.e.,* Δ) between a "high value" and a "low value".

### Nomenclature Table

| **REF. NO.** | **DESCRIPTION** |
|---|---|
| **100** | Photoionization Detector |
| **110** | Housing |
| **119** | Sample Retention Chamber |
| **119₁** | Sample Intake Port |
| **119₂** | Sample Venting Port |
| **120** | UV Lamp |
| **130** | Ignition Electrodes |
| **130₁** | First Ignition Electrode |
| **130₂** | Second Ignition Electrode |
| **140** | Sensing Electrodes 140₁ and 140₂ |
| **140₁** | First Sensing Electrode or Anode |
| **140₂** | Second Sensing Electrode or Cathode |
| **145** | Sensed Signal Amplifier |
| **147** | PID Signal Output Line |
| **150** | Driver for UV Lamp |
| **160** | Ignition Failure Warning Electrical Circuit |
| **160₁** | High Value PID Signal Output Secondary Electrical Signal |
| **160₂** | Low Value PID Signal Output Secondary Electrical Signal |
| **162** | Photodiode |
| **164** | Transimpedance Amplifier |
| **166** | Low Pass Filter |
| **168** | Comparator |
| **169** | Isolation Diode |
| **170** | Processor |
| **180** | Ignition Assist Light Source |
| **A** | Target Analyte |
| **S** | Sample |
| **S_{True}** | Genuine Value Signal Proportional to Target Analyte Concentration |

### Generalized Functional Description

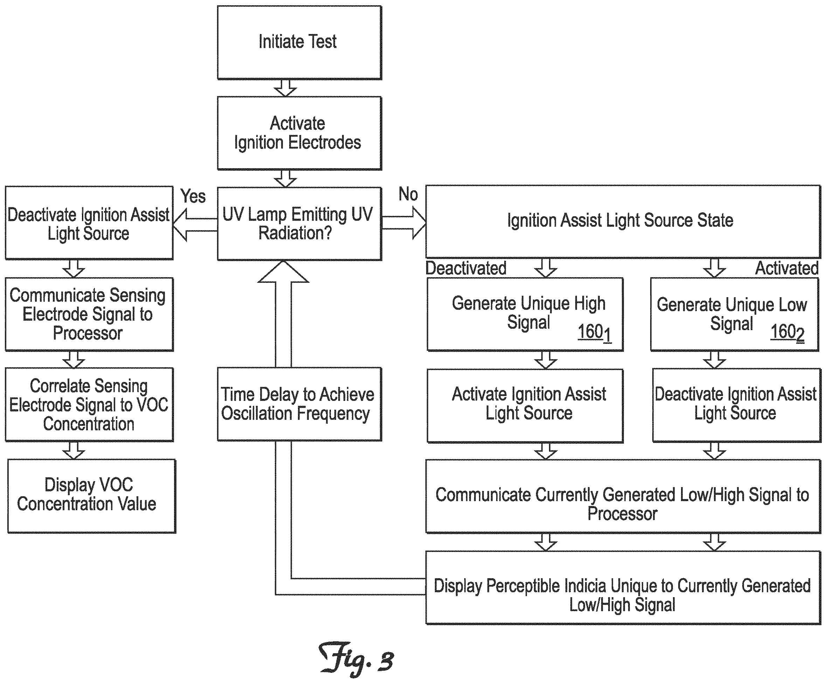

Referring generally to FIG 3, the ignition failure warning circuit **160** provides a human perceptible warning that an attempted ignition of the ultraviolet (UV) lamp **120** was unsuccessful and further ignition attempts are necessary.

In further detail, when the ignition failure warning circuit **160** detects an unlit UV lamp **120,** the ignition failure warning circuit **160** activates the ignition assist light source **180** and upon detecting the emission of light from the ignition assist light source **180** generates and reports a human perceptible higher value PID signal output **160₁**. After a delay selected to allow reporting of changes in the PID signal output as between higher and lower values **160₁** and **160₂** at a human perceptible speed (*e.g*., 0.5 to 2 seconds) the ignition failure warning circuit **160** deactivates the ignition assist light source **180** and upon detecting no emission of light from the ignition assist light source **180** generates a lower value PID signal output **160₂**. If the UV lamp **120** remains unlit, the cycle of ignition assist light source **180** activation and deactivation repeats with the PID signal output and human perceptible reporting continuing to oscillate between the higher and lower values **160₁** and **160₂**. When the UV lamp **120** ignites, the ignition failure warning circuit **160** deactivates the ignition assist light source **180** and releases the PID signal output line **147** to operate normally and report the genuine value PID signal output **S_{True}** which is proportional to the concentration of target analyte **A** in the sample **S** being tested.

### Construction

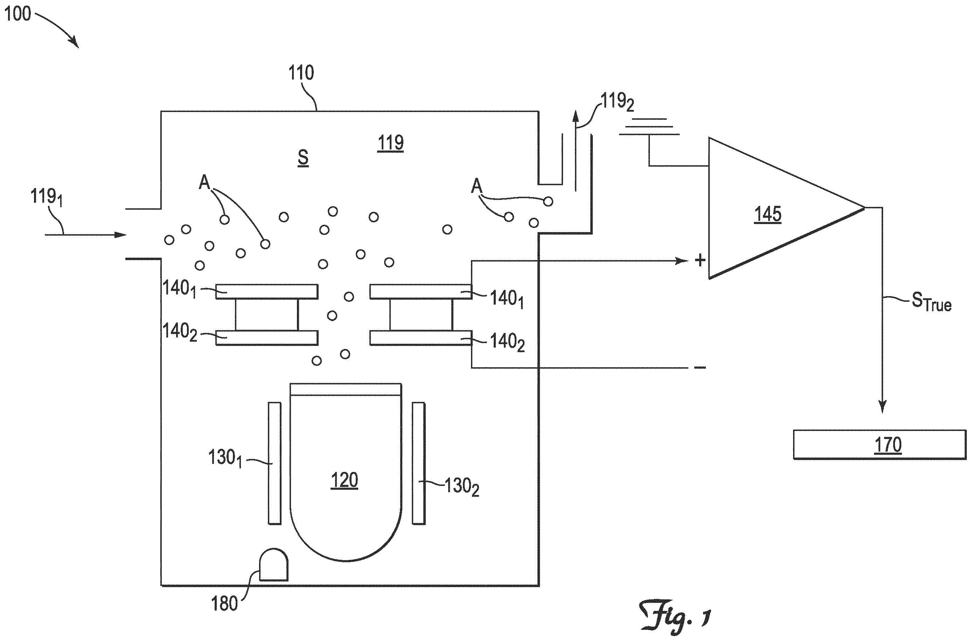

Referring to FIGs 1-2, the invention is directed to a photoionization detector (PID) sensor **100** with an ignition failure warning circuit **160** for indicating a failure of the driver **150** to ignite the ultraviolet (UV) lamp **120.**

Referring to FIG 1, photoionization detector (PID) sensors **100** have an ultraviolet (UV) lamp **120** for ionizing target analyte **A** within a sample **S,** a pair of ignition electrodes **130** (**130₁** and **130₂**) for igniting plasma within the UV lamp **120,** and sensing electrodes **140** *(i.e.,* an anode **140₁** and a cathode **140₂**) for detecting ionized target analyte **A** and generating a genuine value PID electrical current signal **S_{True}** proportional to the concentration of target analyte **A** within the sample **S.** A sensed signal amplifier **145** is typically provided in electrical communication with the sensing electrodes **140** for receiving the generated genuine value PID electrical current signal **S_{True}**, amplifying the signal **S_{True}** and converting the signal **S_{True}** to a voltage electrical signal. These components are generally retained within a housing **110** that defines a sample retention chamber **119** positioned to receive UV radiation emitted by the UV lamp **120** upon excitation of the lamp **120** and having a sample intake port **119₁** and optionally a sample venting port **119₂**.

Photoionization detector (PID) sensors **100** are employed in instruments that typically include a processor **170** for receiving the amplified and converted genuine value PID electrical current signal **S_{True}** and converting the value of the signal to a concentration of target analyte **A** in the sample **S** based upon an algorithm or a lookup table, and displaying or otherwise reporting the target analyte concentration in the sample **S.**

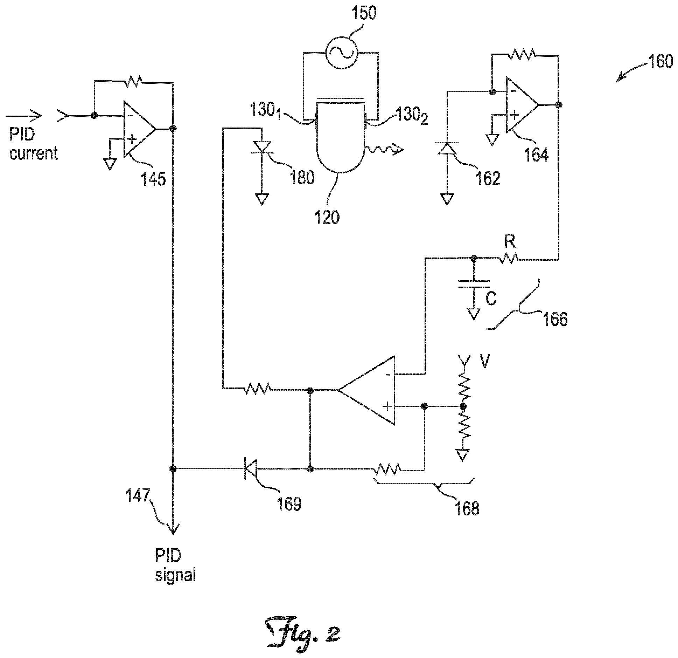

Referring to FIGs 1 and 2, an alternating current is commonly supplied from a power input terminal (unnumbered) through a driver **150** to a pair of ignition electrodes **130₁** and **130₂** positioned on opposite sides of the ultraviolet lamp **120** for igniting the plasma, typically Krypton, retained within ultraviolet lamp **120** and thereby initiating emission of ionizing ultraviolet radiation from the lamp **120.**

Referring to FIG 2, the ignition failure warning circuit **160** alerts a user of the photoionization detector (PID) sensor **100** of an ignition failure by producing and displaying an oscillating signal output of target analyte concentration, preferably at a human perceptible low frequency of approximately 0.1 to 10 Hz, preferably about 0.5 to 5 Hz and most preferably at about 1 Hz, preferably oscillating between zero and full scale, and preferably communicated to the processor **170** along the same output terminal as used to communicate genuine value PID signals **S_{True}** to the processor **170,** whereby the warning is reported as an uncharacteristically dramatic human perceptible oscillation of reported concentrations of target analyte **A.** The ignition failure warning circuit **160** can be powered by the same power input terminal as used to power the ignition electrodes **130.** In a particular embodiment, the PID sensor **100** includes an ignition assist light source **180** and the ignition failure warning circuit **160** also controls activation and deactivation of the ignition assist light source **180.**

Referring generally to FIG 3 and to the embodiment of the invention depicted in FIG 2, the ignition failure warning circuit **160** includes a photodiode **162** capable of detecting visible output from an ignited UV Lamp **120.** The photodiode **162** transmits an electrical signal to a Trans-Impedance amplifier (TIA) **164** where the electrical signal is converted to a voltage, which then in-turn is passed through a low-pass filter or RC network **166** having a time constant, approximately 0.1 to 10 Hz, preferably about 0.5 to 5 Hz and most preferably at about 1 Hz, and then to a comparator **168.** The RC network **166** determines the oscillation rate. The hysteresis of the comparator **168** is adjusted so that it generates and outputs either a high value or a low value voltage dependent upon whether the input voltage value from the comparator **168** is above or below a threshold value selected between the value when the ignition assist light source **180** is activated and the ignition assist light source **180** is deactivated. The output from the comparator **168** stays at a low value as long as the voltage input to the comparator **168** is high (*i.e.,* the UV Lamp **120** is ignited). When the UV lamp **120** is deactivated, then the voltage input to the comparator **168** will decay to a low value and eventually to zero, which effects a transition of the output from the comparator **168** from a low value to a high value, thereby turning on the ignition assist light source **180** and effecting the signal output to a high value by way of the isolation diode **169.**

The photodiode **162** experiences electromagnetic radiation emitted by the ignition assist light source **180.** Hence, when the ignition assist light source **180** is activated (on) the photodiode **162** detects electromagnetic radiation emitted by the ignition assist light source **180,** causing a high value voltage input to the comparator **168,** which in-turn drives the value of the voltage output from the comparator **168** low and deactivates (turns off) the ignition assist light source **180.** This creates an optically-coupled relaxation oscillator with a time base determined by the product of the resistance and capacitance of the RC network **166.**

Proper setting the hysteresis on the comparator **168** will cause oscillation of the reported target analyte **A** concentration value to occur only when the UV lamp **120** is not ignited and not emitting electromagnetic radiation. When the UV lamp **120** is ignited and emitting electromagnetic radiation, photodiode **162** will detect the electromagnetic radiation emitted by the UV lamp **120,** causing the photodiode **162** to transmit a high value voltage input via the transimpedance amplifier **164** and low pass filter **166** to the comparator **168,** which in-turn will drive the value of the voltage output from the comparator **168** low and effect deactivation (turning off) of the ignition assist light source **180.** Thereafter, even with the ignition assist light source **180** deactivated (turned off), the photodiode **162** will continue to detect electromagnetic radiation emitted by the UV lamp **120** and will continue to transmit a high value voltage input via the transimpedance amplifier **164** and low pass filter **166** to the comparator **168,** which in-turn will maintain the voltage output from the comparator **168** low so as to keep the ignition assist light source **180** deactivated (turned off) and discontinue oscillation of target analyte **A** concentration values. This saves power by turning off and keeping off the ignition assist light source **180.** It also pulls the isolation diode **169** low and releases the PID signal output line **147** to transmit genuine value PID signals **S_{True}** generated by the sensing electrodes **140.**

## Claims

1. A photoionization detector with ignition failure warning system, comprising:
(a) an ultraviolet lamp operable when ignited for emitting ultraviolet radiation effective to ionize a target analyte within a sample,
(b) sensing electrodes for detecting the presence of ionized target analyte within the sample and generating a genuine value electrical signal having a value proportional to the concentration of target analyte within the sample,
(c) ignition electrodes for igniting the ultraviolet lamp,
(d) a driver in electrical communication with the ignition electrodes for supplying an ignition current theoretically effective to ignite the ultraviolet lamp,
(e) an ignition failure warning electrical circuit operable for (A) detecting an absence of ultraviolet radiation emitted by the ultraviolet lamp after an attempted ignition of the ultraviolet lamp by the driver, and (B) generating alternating high value and low value secondary electrical signals upon detecting an absence of ultraviolet radiation emitted by the ultraviolet lamp after an attempted ignition of the ultraviolet lamp by the driver, and
(f) a processor for receiving the genuine value electrical signal and the alternating high value and low value secondary signals, and generating a human perceptible indication of failed ignition when the secondary signals are received.

2. The photoionization detector of claim 1 wherein the processor converts the genuine value electrical signal to a corresponding concentration of target analyte when the genuine value electrical signal is received sans receipt of the secondary signals.

3. The photoionization detector of claim 1 wherein the genuine value electrical signal and the secondary electrical signals are communicated to the processor via a common output terminal as indistinguishable signals.

4. The photoionization detector of claim 1 wherein the ignition electrodes and ignition failure warning electrical circuit are powered via a common power input terminal.

5. The photoionization detector of claim 1 wherein the high value and low value secondary electrical signals are reported by the processor as high value and low value concentrations of target analyte respectively, resulting in an uncharacteristically dramatic human perceptible oscillation of reported concentrations of target analyte.

6. The photoionization detector of claim 5 wherein frequency of oscillation is between 0.1 to 10 Hz.

7. The photoionization detector of claim 5 wherein frequency of oscillation is between 0.5 to 5 Hz.

8. The photoionization detector of claim 1 wherein the ultraviolet lamp is operable for ionizing a volatile organic compound target analyte.

9. The photoionization detector of claim 1 further comprising an ignition assist feature wherein a selectively activatable ignition assist light source emits electromagnetic radiation at wavelengths including those at 400 to 500 nm when on and directs the electromagnetic radiation at the ultraviolet lamp for assisting ignition of the ultraviolet lamp by the ignition electrodes.

10. The photoionization detector of claim 9 wherein the ignition failure warning electrical circuit is further operable for turning off the selectively activatable ignition assist light source upon detecting presence of ultraviolet radiation emitted by the ultraviolet lamp after an attempted ignition of the ultraviolet lamp, until at least a subsequent attempt to ignite the ultraviolet lamp with the ignition electrodes.

11. The photoionization detector of claim 1, wherein the ignition failure warning electrical circuit comprises:
(a) a photodiode operable for (A) periodically detecting presence and absence of radiation emitted by at least one of the ultraviolet lamp or the ignition assist light source after an attempted ignition of the ultraviolet lamp by the driver, and (B) periodically generating an ON current signal when the presence of ultraviolet radiation emitted by at least one of the ultraviolet lamp and the ignition assist light source is detected, and otherwise bypassing generation of an ON signal,
(b) a transimpedance amplifier in electrical communication with the photodiode operable for receiving and converting each ON current signal to an ON voltage signal and in the absence of receiving an ON current signal bypassing generation of an ON voltage signal,
(c) a low pass filter having a time constant between 0.1 to 10 Hz in electrical communication between the transimpedance amplifier, the ignition assist light source and the processor for receiving each ON voltage signal and providing a timed passage of each ON voltage signal in accordance with the time constant, and
(d) a comparator with hysteresis in electrical communication with the low pass filter for receiving each ON voltage signal and generating a first electrical output when each ON voltage signal is received operable for powering off the ignition assist light source and transmitting the low value secondary electrical signal to the processor, and generating a second electrical output when no ON voltage signal is received operable for powering on the ignition assist light source and transmitting the high value secondary electrical signal to the processor.

12. The photoionization detector of claim 1 further comprising a housing defining a sample retention chamber and wherein the ultraviolet lamp is operable when ignited for emitting ultraviolet radiation effective to ionize a target analyte within a sample retained within the sample retention chamber.

13. The photoionization detector of claim 12 wherein the sensing electrodes are an anode-cathode pair for detecting the presence of ionized target analyte within the sample retention chamber.

14. The photoionization detector of claim 1 further comprising a selectively activatable ignition assist light source different from the ultraviolet lamp for emitting electromagnetic radiation ineffective for detectably ionizing a target analyte within a sample, wherein the ignition failure warning electrical circuit is operable for generating alternating high value and low value secondary electrical signals upon detecting an absence of ultraviolet radiation emitted by the ultraviolet lamp after an attempted ignition of the ultraviolet lamp by the driver by (i) oscillating powering of the ignition assist light source as between a light-on status and light-off status upon detecting an absence of ultraviolet radiation emitted by the ultraviolet lamp after an attempted ignition of the ultraviolet lamp by the driver, (ii) detecting the on-off status of the ignition assist light source over time, and (iii) generating a light-on electrical signal having a value when the ignition assist light source is on and generating a light-off electrical signal having a value different from the value of the light-on electrical signal when the ignition assist light source is off, so as to generate an oscillating set of high value and low value secondary electrical signals.
